# EUROPEAN PATENT APPLICATION

(11) **EP 2 868 337 A1**
(43) Date of publication of application: **06.05.2015**
(21) Application number: 14188974.1
(22) Date of filing: 15.10.2014
(51) Int. Cl.: A61M 5/168

(54) **Fluid transport apparatus, fluid infusing apparatus, and trouble determining method for transporting tube**

(30) Priority: 17.10.2013 JP 2013216104; 22.01.2014 JP 2014009252
(71) Applicant: Seiko Epson Corporation, Shinjuku-ku, Tokyo 163-0811 (JP)
(72) Inventor: Katase, Makoto, Suwa-shi Nagano, 392-8502 (JP)
(74) Representative: Hoffmann Eitle

(57) **Abstract**

A fluid transport apparatus (10) includes: a first electrode (36) provided on the insulating transporting tube which constitutes part of a flow channel in which conductive fluid is transported, and configured not to come into contact with the fluid in the flow channel a second electrode (26) configured to come into contact with fluid in the flow channel; and a determining unit connected to the first electrode and the second electrode, and configured to determine a trouble of the transporting tube. Accordingly, the trouble of the transporting tube is determined.

## Description

### BACKGROUND

### 1. Technical Field

The present invention relates to a fluid transport apparatus, a fluid infusing apparatus, and a trouble determining method for a transporting tube configured to transport fluid.

### 2. Related Art

As a fluid transport apparatus, for example, a micro pump including a plurality of fingers disposed between a tube that transports fluid and a cam, and configured to transport the fluid in the tube by compressing the tube with the plurality of fingers in sequence by pressing the plurality of fingers with a rotating cam as described in JP-A-2010-77947 is known.

In order to infuse fluid such as insulin into a biological body, by providing a catheter indwelled in the biological body in the fluid transport apparatus as described above, regular infusion of the fluid into the biological body is achieved. However, the regular infusion of the fluid into the biological body is disabled unless troubles such as disconnection or clogging of the catheter or the tube that transports the fluid are not detected.

### SUMMARY

An advantage of some aspects of the invention is to provide a fluid transport apparatus, a fluid infusing apparatus, and a trouble determining method for a transporting tube (catheter or tube) capable of determining trouble of the transporting tube.

One embodiment for achieving the advantage is directed to a fluid transporting apparatus including: an insulative transporting tube that constitutes part of a flow channel in which a conductive fluid is transported; a first electrode provided on the transporting tube and configured not to come into contact with the fluid in the flow channel; and a second electrode configured to come into contact with the fluid in the flow channel.

Other characteristics of the invention will be apparent from the specification and attached drawings.

### BRIEF DESCRIPTION OF DRAWINGS

The invention will be described with reference to the accompanying drawings, wherein like numbers reference like elements.
Fig. 1A is an exploded view of a fluid transport apparatus of a first embodiment. Fig. 1B is an explanatory drawing of a pump unit.
Fig. 2 is a cross-sectional view of the fluid transport apparatus.
Fig. 3A is an explanatory drawing of a catheter of the first embodiment. Fig. 3B is a trouble determining method for the catheter.
Fig. 4 is an explanatory drawing of a catheter of a second embodiment.
Fig. 5 is an explanatory drawing of a catheter of a third embodiment.
Fig. 6A is an explanatory drawing of a fluid transport apparatus of a fourth embodiment. Fig. 6B is a cross-sectional view of a fluid infusing portion.
Fig. 7 is an explanatory drawing of a trouble determining method for the catheter and the connecting tube of the fourth embodiment.
Fig. 8 is an explanatory drawing of a trouble determining method for the catheter and the connecting tube of a fifth embodiment.
Fig. 9 is an explanatory drawing of a trouble determining method for the catheter of a sixth embodiment.
Fig. 10 is an explanatory drawing of a fluid transport apparatus of a seventh embodiment.
Fig. 11 is an explanatory drawing of a trouble determining method for a catheter 31 of the seventh embodiment.
Fig. 12 is an explanatory drawing of a fluid transport apparatus of an eighth embodiment.
Fig. 13 is an explanatory drawing of a trouble determining method for the catheter 31 and a connecting tube 80 of the eighth embodiment.
Fig. 14 is an explanatory drawing of a trouble determining method for a catheter 71 and the connecting tube 80 of a ninth embodiment.

### DESCRIPTION OF EXEMPLARY EMBODIMENTS

According to the description of the specification and the attached drawings, at least the followings become apparent.

A fluid transporting apparatus may include an insulative transporting tube which constitutes part of a flow channel in which conductive fluid is transported; a first electrode provided on the transporting tube and configured not to come into contact with the fluid in the flow channel; and a second electrode configured to come into contact with fluid in the flow channel; a potential measuring unit configured to measure a potential between the first electrode and the second electrode, and a determining unit configured to determine trouble of the transporting tube on the basis of the potential measured by the potential measuring unit.

According to the fluid transport apparatus configured as described above, a current path configured to close between the first electrode and the second electrode via fluid or a biological body is formed within a predetermined potential range if the transporting tube is normal, and the current path configured to close between the first electrode and the second electrode changes if the transporting tube has a trouble, so that a trouble of the transporting tube may be determined on the basis of the potential between the electrodes.

In the fluid transport apparatus as described above, the transporting tube may be a catheter configured to be inserted into the biological body in order to infuse the fluid into the biological body.

According to the fluid transport apparatus configured as described above, the current path configured to close between the first electrode and the second electrode via the fluid or the biological body is formed within the predetermined potential range if the transporting tube is normal, and the current path configured to close the first electrode and the second electrode changes if the transporting tube has a trouble, so that the trouble of the transporting tube can be determined on the basis of the potential between the electrodes.

In the fluid transport apparatus as described above, the catheter may be provided with the first electrode on an outer peripheral surface thereof at a portion other than an end portion on a side to be inserted into the biological body.

According to the fluid transport apparatus configured as described above, contact of the first electrode to a fluid trap generated at a position of the catheter pulled out from the biological body can be restrained, so that the trouble of the transporting tube can be determined further accurately.

In the fluid transport apparatus as described above, the transporting tube may be a connecting tube configured to connect a fluid storage portion in which the fluid is stored and a fluid infusing portion provided with the catheter to be inserted into the biological body for infusing the fluid into the biological body.

According to the fluid transport apparatus configured as described above, the current path configured to close between the first electrode and the second electrode via the fluid or the biological body is formed within the predetermined potential range if the transporting tube is normal, and the current path configured to close the first electrode and the second electrode changes if the transporting tube has a trouble, so that the trouble of the transporting tube can be determined on the basis of the potential between the electrodes.

In the fluid transport apparatus as described above, the second electrode may be a conductive film provided on an inner peripheral surface of the transporting tube or a conductive tube connected to the transporting tube and which constitutes part of the flow channel.

According to the fluid transport apparatus configured as described above, the fluid in the flow channel can be brought into contact with the second electrode.

In the fluid transport apparatus as described above, the first electrode may be provided on the outer peripheral surface of the transporting tube, part of the outer peripheral surface of the transporting tube may be exposed, and the fluid flowing in the interior of the transporting tube may be visible.

According to the fluid transport apparatus configured as described above, the first electrode can be provided on the transporting tube so as to avoid contact with the fluid in the flow channel, and the state of the fluid flowing in the interior of the transporting tube (the position of the fluid or whether or not air bubbles are generated) can be confirmed.

In the fluid transport apparatus as described above, the first electrode may be embedded in the transporting tube.

According to the fluid transport apparatus configured as described above, noise caused by the first electrode by coming into contact with the conductive member provided outside, so that the trouble of the transporting tube can be accurately determined further accurately.

In the fluid transport apparatus as described above, the flow channel is preferably provided with a pump configured to cause the fluid to flow.

In this configuration, the fluid can be caused to flow in the flow channel.

In the fluid transport apparatus as described above, the pump preferably causes the fluid to flow by compressing the tube in which fluid flows with a plurality of pressing members.

In this configuration, the fluid can be caused to flow in the flow channel adequately.

In the fluid transport apparatus as described above, the pump may cause the fluid to flow by changing the capacity of a container in which the fluid is stored.

In this configuration as well, the fluid can be caused to flow in the flow channel adequately.

A trouble determining method for a transporting tube includes: measuring a potential between a first electrode provided on an insulative transporting tube which constitutes part of a flow channel in which conductive fluid is transported and configured not to come into contact with the fluid in the flow channel and a second electrode configured to come into contact with the fluid in the flow channel; and determining a trouble of the transporting tube on the basis of the measured potential.

According to the fluid transport apparatus configured as described above, a current path configured to close between the first electrode and the second electrode via the fluid or a biological body is formed within a predetermined potential range if the transporting tube is normal, and the current path configured to close the first electrode and the second electrode changes if the transporting tube has a trouble, so that the trouble of the transporting tube can be determined on the basis of the potential between the electrodes.

### First Embodiment

### Basic Configuration of Fluid Transport Apparatus 1

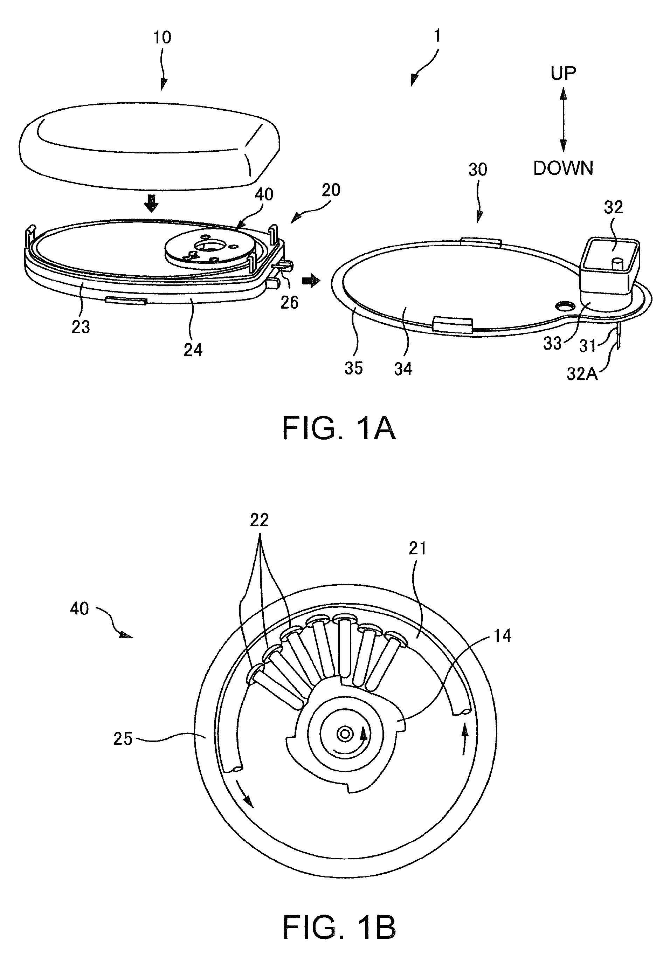

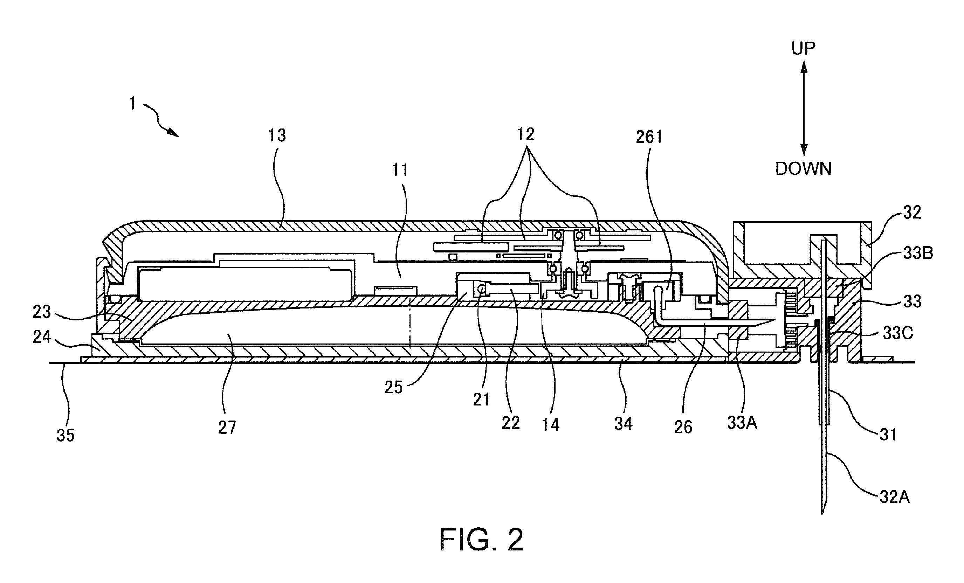

Fig. 1A is an exploded view of a fluid transport apparatus 1 of a first embodiment. Fig. 1B is an explanatory drawing of a pump unit 40. Fig. 2 is a cross-sectional view of the fluid transport apparatus 1. For the convenience of description, a side where the fluid transport apparatus 1 is adhered to a biological body is defined as a lower side, and an opposite side is referred to as an upper side.

The fluid transport apparatus 1 of the first embodiment includes a main body portion 10, a cartridge 20, and a patch portion 30 as illustrated in Fig. 1A. The main body portion 10, the cartridge 20, and the patch portion 30 are separable as illustrated in Fig. 1A, and are assembled integrally when in use. The fluid transport apparatus 1 is configured to be adhered to a biological body at a lower surface of the patch portion 30, and is used for infusing fluid stored in the cartridge 20, for example, drug solution such as insulin into the biological body. The fluid transported by the fluid transport apparatus 1 is not limited to the drug solution, and may be other liquid or fluid in the form of sol or gel.

The main body portion 10 is configured to transport fluid stored in the cartridge 20 to the patch portion 30 and, as illustrated in Fig. 2, includes a main body base 11, a drive mechanism 12 and a control unit (a control substrate which is not illustrated) provided on the main body base 11, a main body case 13 configured to cover the drive mechanism 12 and the control unit, and a cam 14 projecting downward from the main body base 11. The drive mechanism 12 is a mechanism for rotating the cam 14. In the drive mechanism 12, for example, a rotor is rotated by a piezoelectric motor, and the rotation of the rotor is transmitted to the cam 14 at a predetermined reduction ratio by a deceleration mechanism.

The cartridge 20 is a tube configured to transport fluid and includes a resilient pump tube 21, a plurality of fingers 22 (seven fingers in this case) that push the pump tube 21, a cartridge base 23, a base receiver 24, a pump base 25 provided on the cartridge base 23, and a connecting needle 26, as illustrated in Figs. 1A and 1B and Fig. 2. A storage portion 27, which is a space for storing fluid, is defined between the cartridge base 23 and the base receiver 24.

As illustrated in Fig. 1B, the pump tube 21 is arranged along a side wall inside the bottomed cylindrical pump base 25, and an end portion of the pump tube 21 on an upstream side (upstream side in the direction of fluid transport) communicates with the interior of the storage portion 27 via a joint (not illustrated), and an end portion of the pump tube 21 on a downstream side communicates with the interior of the connecting needle 26 via a joint 261. The cam 14 projecting downward from the main body base 11 is arranged at a center portion of the pump base 25, and the plurality of fingers 22 are arranged between the pump tube 21 and the cam 14. The cam 14 includes a plurality (four in this case) of projecting portions on an outer periphery thereof, and when the cam 14 rotates, the plurality of fingers 22 are pressed from the upstream side in the direction of fluid transport by the projecting portions of the cam 14 in sequence. The fingers 22 pressed by the cam 14 compress the pump tube 21, and the fluid in the interior of the pump tube 21 is transported in the direction of fluid transport. In other words, the pump tube 21, the fingers 22, and the pump base 25 on the cartridge 20 side and the drive mechanism 12 and the cam 14 on the main body portion 10 side constitute the pump unit 40, and the fluid in the interior of the storage portion 27 is transported to the pump tube 21, the connecting needle 26, and the patch portion 30 in this order by the pump unit 40. A configuration of the pump unit 40 is not limited to the configuration described above, as long as the fluid in the interior of the storage portion 27 can be transported to the patch portion 30.

The patch portion 30 includes a catheter 31, an introduction needle folder 32, a port base 33, a patch base 34, and an adhesive pad 35 as illustrated in Fig. 1A. The patch base 34 is a flat panel-shaped member fixed to the port base 33, and configured to cover a lower surface of the cartridge 20. The adhesive pad 35 is mounted on a lower surface of the patch base 34, and the fluid transport apparatus 1 is adhered to the biological body by the adhesive pad 35.

The catheter 31 is a relatively soft tube which is inserted into and indwelled in the biological body for infusing the fluid into the biological body. The introduction needle folder 32 is a member configured to fix an upper end of an introduction needle 32A and retaining the introduction needle 32A as illustrated in Fig. 2. The introduction needle 32A is a metallic needle for inserting the soft catheter 31 into the biological body. When the fluid transport apparatus 1 is mounted on the biological body, the introduction needle folder 32 is attached to the port base 33, and the introduction needle 32A is passed through the catheter 31 so that a needle point projects from the catheter 31. After the introduction needle 32A has been inserted into the biological body together with the catheter 31, the introduction needle folder 32 is pulled out from the port base 33 together with the introduction needle 32A. Accordingly, the hard introduction needle 32A does not stay in the biological body, and only the soft catheter 31 stays in the biological body, so that a burden on the biological body may be reduced.

A connecting needle septum 33A for passing the connecting needle 26 on the cartridge 20 side is provided on a side portion of the port base 33 (see Fig. 2), and an introduction needle septum 33B configured to pass the introduction needle 32A is provided on an upper portion of the port base 33. The septums 33A and 33B are formed of a material which closes holes when the needles 26 and 32A are pulled out where the needles 26 and 32A are passed (for example rubber or silicon). A coupling portion 33C is provided on a lower portion of the port base 33, and the catheter 31 is coupled to a lower end of the coupling portion 33C. The coupling portion 33C is provided with a hole for passing the introduction needle 32A therethrough, and the hole communicates with the interior of the catheter 31, and after the introduction needle 32A has been pulled out, the hole serves as a flow channel of the fluid. The catheter 31 may be extended and coupled directly to the port base 33 without providing the coupling portion 33C.

### Trouble Determining Method for Catheter 31

After the fluid transport apparatus 1 has been mounted on the user, the control unit provided on the main body portion 10 controls the pump unit 40 in accordance with a setting of the user, and controls an amount of fluid transport per unit time, or controls fluid so as to be transported at every predetermined period. Accordingly, an adequate amount of fluid is regularly infused into the biological body. However, there are the cases that the catheter 31 picked into the biological body comes away, the catheter 31 is broken, or foreign particles enter and clog the catheter at the moment when the user moves. Consequently, even though the fluid transport apparatus 1 transports the fluid, the fluid cannot be infused into the biological body. In particular, since the catheter 31 is generally formed of a soft member in order to alleviate the load on the biological body, the catheter 31 may come away easily in comparison with a metallic needle such as the introduction needle 32A. Also, by reducing the length of the catheter 31 in order to alleviate the load on the biological body, the catheter 31 may come away easily. Therefore, in the fluid transport apparatus 1 of the first embodiment, a process of determining a trouble such as coming away from the biological body or clogging of the catheter 31 is performed. For example, a trouble determining process is performed on the catheter 31 regularly in accordance with the setting of the user or the like.

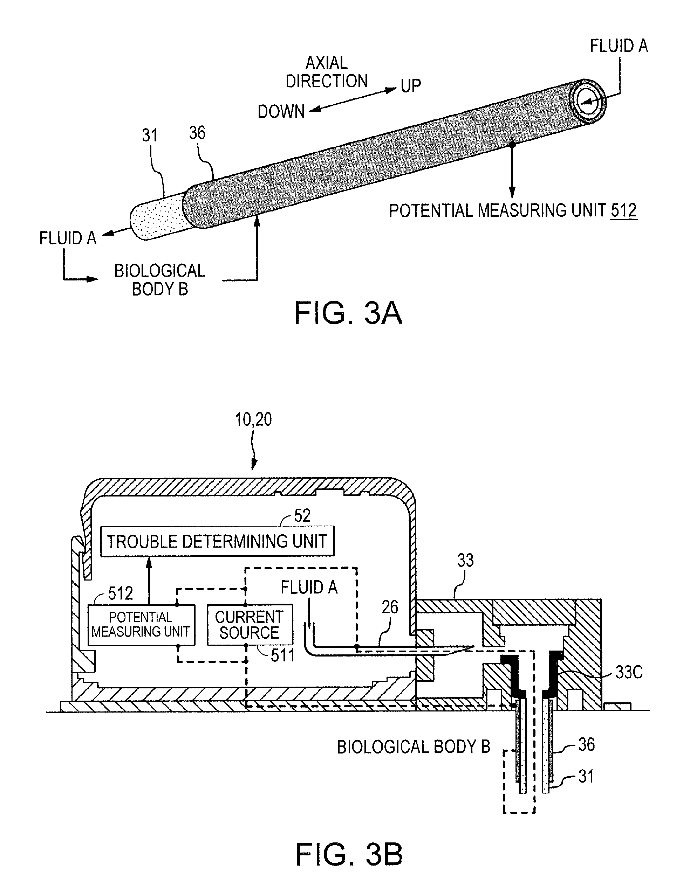

Fig. 3A is an explanatory drawing of the catheter 31 of the first embodiment. Fig. 3B is an explanatory drawing of a trouble determining method for the catheter 31. Fluid A which is transported by the fluid transport apparatus 1 is assumed to be a conductive fluid (for example, drug solution including normal saline solution) for the trouble determining process. As illustrated in Fig. 3A, an electrode member 36 (first electrode) is provided over an entire circumferential of an outer peripheral surface of the insulative catheter 31, and the electrode member 36 is configured not to come into contact with the fluid flowing in the interior of the catheter 31. In addition, the electrode member 36 is configured not to come into contact with the fluid flowing in the interior of the port base 33 by the coupling portion 33C. Examples of the catheter 31 include those formed of a soft material such as a fluorine contained resin or polyurethane, and examples of the electrode member 36 include those formed of a metal such as titan which has least impact on human bodies. In order to increase adhesiveness between the catheter 31 and the electrode member 36, a resin which constitutes the catheter 31 and a resin member in which a conductive filler kneaded or a metallic member may be molded integrally by an extrusion processing.

Then, an electrode which pairs with the electrode member 36 provided on the catheter 31 is determined as an electrode which comes into contact with the fluid (second electrode), and, in the first embodiment, the connecting needle 26 provided on the cartridge 20 is determined to be the second electrode. In order to do so, the connecting needle 26 formed entirely of a conductive member such as a metal or formed of a conductive member on an inner peripheral surface thereof that comes into contact with the fluid flowing in the interior of the connecting needle 26 is employed. The connecting needle 26 and the electrode member 36 are arranged at positions separate from each other and are insulated.

In addition, the control unit provided on the main body portion 10 is provided with a current source 511, a potential measuring unit 512, and a trouble determining unit 52 as illustrated in Fig. 3B. The current source 511 is electrically connected to the electrode member 36 provided on the catheter 31, and also electrically connected to the connecting needle 26 (portion conducting with the fluid flowing in the interior), and supplies constant current to the connecting needle 26 side. The potential measuring unit 512 is electrically connected between the electrode member 36 and the current source 511, and between the connecting needle 26 and the current source 511, whereby a potential between the electrode member 36 and the connecting needle 26 is measured. It is also possible to configure the current source 511 to supply a constant current to the electrode member 36 side.

Then, in the case where the catheter 31 does not come away from the biological body or is not clogged, that is, when the catheter 31 is normal, the current source 511, the connecting needle 26, the fluid A flowing in the interiors of the connecting needle 26, the port base 33, and the catheter 31, a biological body B, and the electrode member 36 constitute a closed current path, and a predetermined current flows therethrough as illustrated in Fig. 3B. Therefore, a potential within a predetermined range in accordance with the current from the current source 511 is measured. In contrast, in the case where the catheter 31 has a trouble such that the catheter 31 comes away from the biological body or is clogged, the closed current path via the fluid A flowing in the interior of the catheter 31 and the biological body B may not be formed, or the predetermined current may not be flowed to the electrode member 36. In other words, the fluid A and the electrode member 36 are insulated by the catheter 31, for example, and a potential which is deviated from a potential estimated in the case where the catheter 31 is normal is measured. For example, a potential which is not different from the case where the current source 511 does not supply a current is measured.

Therefore, for example, when a potential within the predetermined range is measured on the basis of a result of measurement of the potential measuring unit 512, the trouble determining unit 52 determines that the catheter 31 is normal, and when a potential deviated from the predetermined range is measured, the trouble determining unit 52 determines that the catheter 31 has a trouble, and outputs these results of determination to the control unit. When the catheter 31 is normal, the control unit continues a fluid transporting process, and when the catheter 31 has a trouble, the control unit stops the fluid transporting process, and notifies the trouble of the catheter 31 to the user by sound or light. In this process, the user who has notified the trouble of the catheter 31 is allowed to perform a process such as re-insert the catheter 31 or the like, so that the fluid may be infused into the biological body again.

As described thus far, the fluid transport apparatus 1 of the first embodiment includes the insulative catheter 31 (transporting tube) which constitutes part of the flow channel in which the conductive fluid is transported; the electrode member 36 (first electrode) provided on the catheter 31 and does not come into contact with the fluid in the flow channel; the connecting needle 26 (second electrode) configured to come into contact with the fluid in the flow channel; the potential measuring unit 512 configured to measure the potential between the electrode member 36 and the connecting needle 26, and the trouble determining unit 52 (determining unit) configured to detect the trouble of the catheter 31 on the basis of the potential obtained by the potential measuring unit 512. In the fluid transport apparatus 1 having the configuration as described above, if the catheter 31 is normal, the current path which closes between the electrode member 36 and the connecting needle 26 via the fluid and the biological body is formed, while if the catheter 31 has a trouble, the current path which closes a connection between the electrode member 36 and the connecting needle 26 changes. Therefore, the trouble of the catheter 31 may be determined on the basis of the potential between the electrode member 36 and the connecting needle 26. The flow channel in which the fluid is transported is a flow channel through which the fluid flows from the storage portion 27 of the cartridge 20 through the pump tube 21, the connecting needle 26, the port base 33, and the catheter 31 in this order.

The flow channel of the fluid transport apparatus 1 of the embodiment used for administration of insulin or the like is not a circulating flow channel, but a single flow channel from the storage portion 27 to the catheter 31. However, in the embodiment, the electrode member 36 is provided on the catheter 31 to be inserted into the biological body as a part of the current path. Therefore, the closed current path may be formed without providing another flow channel. Not only when the catheter 31 has a trouble, but also when the flow channel of the fluid flowing from the storage portion 27 to the catheter 31 is clogged in the midcourse, the closed current path changes and hence it is determined that there is a trouble. In other words, not only the trouble of the catheter 31, but also the trouble of the entire flow channel can be determined.

Since the connecting needle 26, which is an electrode which pairs with the electrode member 36 comes into contact with the fluid flowing in the interior thereof, a measurement error of the potential is alleviated and an accuracy of determination is improved in comparison with the case where the electrode does not come into direct contact with the fluid. Since the connecting needle 26 is used as the electrode that pairs with the electrode member 36 without providing another member, an increase in number of components may be prevented. However, the invention is not limited thereto, and an electrode configured to come into contact with fluid may be provided on the joint 261 of the connecting needle 26 or the pump tube 21, for example.

The electrode member 36 is provided at a portion of the outer peripheral surface of the catheter 31 other than an end portion on the side inserted into the biological body (lower side), and a lower end portion of the catheter 31 is exposed. Therefore, contact of the electrode member 36 to a fluid trap generated at a position of the catheter 31 pulled out from the biological body is restrained. In other words, such an event that the electrode member 36 comes into contact with the fluid trap and hence the catheter 31 is determined to be normal even though the catheter 31 is disconnected may be restrained, and hence the trouble of the catheter 31 is determined further accurately. The catheter 31 is formed of a transparent or opaque member, and the fluid flowing in the interior of the catheter 31 is visible from an exposed outer peripheral surface of the catheter 31. In this configuration, the state of the fluid in the interior of the catheter 31 (for example, the position of the fluid or whether or not air bubbles are generated) can be confirmed. However, the invention is not limited thereto and, for example, the entire outer peripheral surface of the catheter 31 may be covered with the electrode member 36 or a portion other than the lower end portion of the catheter 31 may be exposed or, alternatively, the catheter 31 may be formed of a non-transparent member.

Alternatively, it is assumed that the electrode member 36 is provided only at an end portion of the outer peripheral surface of the catheter 31 on a side opposite to the side (upper side) that is inserted into the biological body. In this case, even though the catheter 31 slightly moves away from the biological body, the electrode member 36 does not come into contact with the biological body, and it is determined that there is a trouble. In contrast, according to the embodiment, the electrode member 36 extends in a direction of axis of the catheter 31. Therefore, the electrode member 36 is kept in contact with the biological body until the catheter 31 comes away from the biological body to an extent that the fluid cannot be infused from the catheter 31 into the biological body, so that being determined as a trouble is prevented. Therefore, the trouble of the catheter 31 is further accurately determined. However, the invention is not limited thereto, and the electrode member 36 may be provided only on an upper end portion of the outer peripheral surface of the catheter 31.

### Second Embodiment

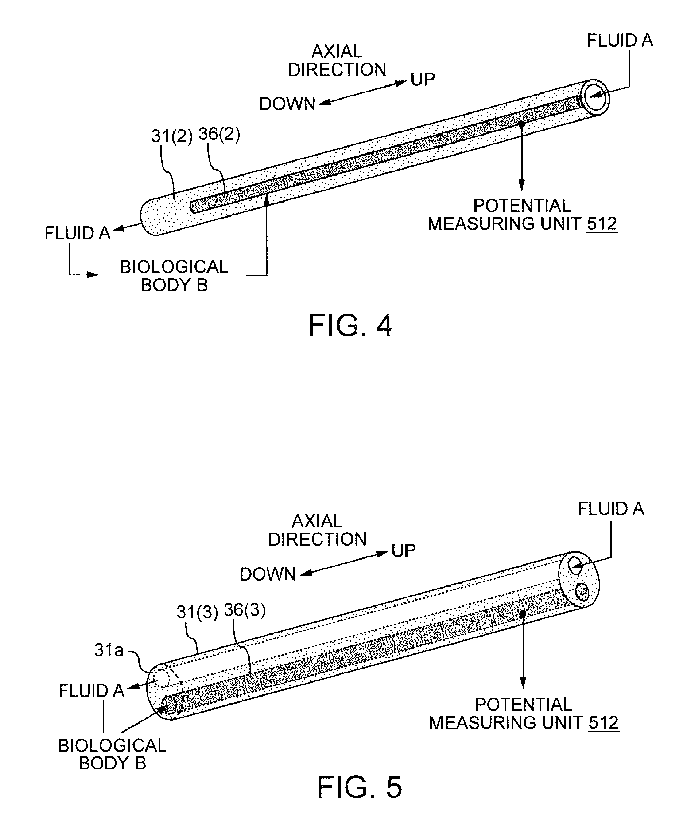

Fig. 4 is an explanatory drawing of a catheter 31 (2) of a second embodiment. In the second embodiment, only the configuration of the catheter is different from the first embodiment, and the width of an electrode member 36 (2) in a direction orthogonal to the axial direction of the catheter 31 (2) is short, and the electrode member 36 (2) is provided only on part of an outer peripheral surface of the catheter 31 (2) in the circumferential direction. Therefore, the outer peripheral surface of the catheter 31 (2) is exposed from an upper end to a lower end, and a range of visibility of a fluid flowing in the interior of the catheter 31 (2) is increased. Therefore, the state of the fluid in the interior of the catheter 31 (2) is confirmed further easily. In the same manner as the first embodiment, in the catheter 31 (2) of the second embodiment as well, the electrode member 36 is not provided on a lower end portion, and the electrode member 36 (2) extends in the axial direction. Therefore, the trouble of the catheter 31 is determined further accurately.

### Third Embodiment

Fig. 5 is an explanatory drawing of a catheter 31 (3) of a third embodiment. In the third embodiment, only the configuration of the catheter is different from the first embodiment, and an electrode member 36 (3) is embedded in the catheter 31 (3) so that the electrode member 36 (3) does not come into contact with fluid flowing in the interior of the catheter 31 (3). The electrode member 36 (3) is exposed from an end surface 31a of the catheter 31 (3) on the side to be inserted into the biological body (lower side) so that the electrode member 36 (3) comes into contact with the fluid and the biological body further reliably. In this case as well, as illustrated in Fig. 5, since the current from the connecting needle 26 flows into the electrode member 36 (3) via the fluid A and the biological body B, the trouble of the catheter 31 (3) may be determined. Since the outer peripheral surface (other than end surfaces in the axial direction) of the electrode member 36 (3) is covered with the catheter 31 (3), generation of noise caused by contact between the electrode member 36 (3) and the conductive member provided outside may be restrained, and a trouble of the catheter 31 (3) may be determined further accurately. In addition, since a contact area between the electrode member 36 (3) and the biological body is small, an influence on the biological body may be reduced.

### Fourth Embodiment

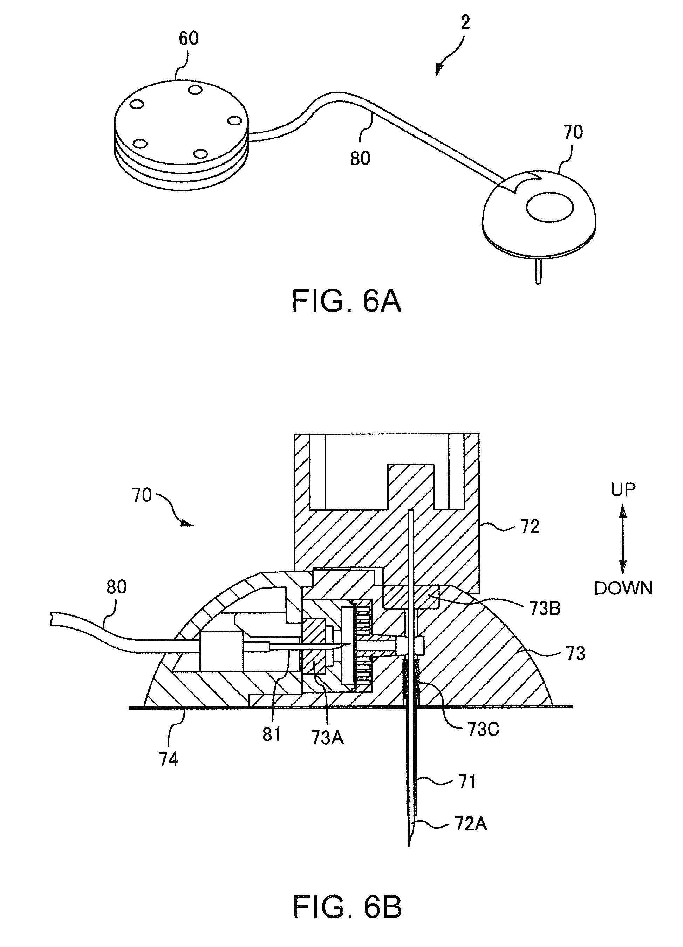

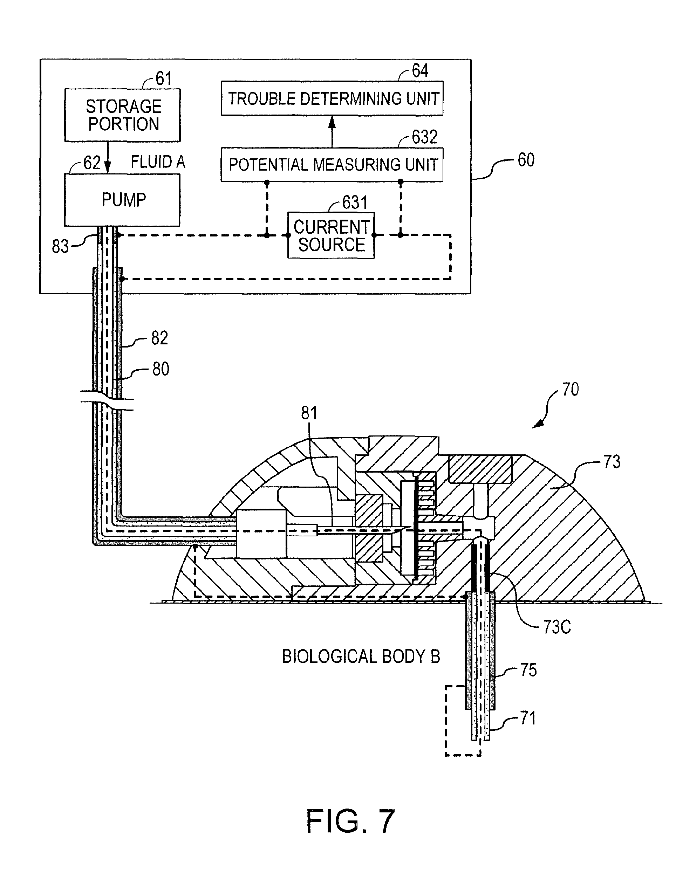

Fig. 6A is an explanatory drawing of a fluid transport apparatus 2 of a fourth embodiment. Fig. 6B is a cross-sectional view of a fluid infusing portion 70. Fig. 7 is an explanatory drawing of a trouble determining method for a catheter 71 and a connecting tube 80 of the fourth embodiment. In the fourth embodiment, as illustrated in Fig. 6A, the fluid transport apparatus 2 including a main body portion 60 (fluid storage portion) in which the fluid is stored, that is, the main body portion 60 attached to clothes or the like of the user, and the fluid infusing portion 70 adhered to the biological body connected to each other with a flexible elongated connecting tube 80 (transporting tube) will be exemplified. The main body portion 60 includes a storage portion 61 configured to store fluid therein, a pump 62 configured to transport the fluid in the storage portion 61 to the fluid infusing portion 70 by the connecting tube 80, a current source 631, a potential measuring portion 632, and a trouble determining unit 64 as illustrated in Fig. 7. The fluid infusing portion 70 includes the catheter 71, an introduction needle folder 72, an introduction needle 72A, a port base 73, and an adhesive pad 74 as illustrated in Fig. 6B. Then, a septum 73A configured to pass a connecting needle 81 coupled to an end portion of the connecting tube 80 is provided on the side portion of the port base 73, a septum 73B configured to pass the introduction needle 72A is provided on an upper portion of the port base 73, and a coupling portion 73C configured to couple the catheter 71 to a lower end is provided on a lower portion of the port base 73. The coupling portion 73C is provided with a hole for passing the introduction needle 72A therethrough, and the hole serves as a flow channel of the fluid after the introduction needle 72A has been pulled out. The connecting tube 80 is formed of an insulative member (for example, a fluorine contained resin or polyurethane) in the same manner as the catheter 71.

In the case of the fluid transport apparatus 2 of the fourth embodiment, not only the catheter 71 may come away from the biological body or may be clogged, but also the connecting tube 80 may come away from the main body portion 60 or the fluid infusing portion 70, or may be clogged due to bending. Accordingly, even though the fluid transport apparatus 2 transports fluid, the fluid cannot be infused into the biological body. Therefore, in the fluid transport apparatus 2 of the fourth embodiment, a process of determining a trouble such as coming away of the catheter 71 and the connecting tube 80 or clogging is performed.

Therefore, electrode members 75 and 82 (first electrode) which do not come into contact with the fluid in the flow channel are provided on the catheter 71 and the connecting tube 80 respectively. Examples of a method of disposing the electrode members 75 and 82 include a method of disposing the electrode member 36 on the catheter 31 in the first to the third embodiments described above. The method of disposing the electrode member 75 on the catheter 71 and the method of disposing the electrode member 82 on the connecting tube 80 may be and may not be the same. The electrode member 75 of the catheter 71 and the electrode member 82 of the connecting tube 80 are electrically connected.

Furthermore, an electrode tube 83 (second electrode), which is a conductive tube (for example, a metallic tube) which constitutes part of the flow channel of the fluid flowing from the storage portion 61 to the catheter 71 is connected to an end portion of the connecting tube 80 on the main body portion 60 side as electrodes which pair with the electrode members 75 and 82 provided on the catheter 71 and the connecting tube 80 and come into contact with the fluid. Since the electrode tube 83 comes into contact with the fluid flowing in the interior of the electrode tube 83, the measurement error of the potential may be alleviated in comparison with the case where the electrode does not come into direct contact with the fluid. The electrode tube 83 is arranged at a position that does not come into contact with the electrode member 82 provided on the connecting tube 80 and is insulated from the electrode member 82. The electrode member 82 of the connecting tube 80 electrically connected to the electrode member 75 of the catheter 71 and the current source 631 are electrically connected and the electrode tube 83 and the current source 631 are electrically connected. The potential measuring portion 632 is electrically connected between the electrode member 82 and the current source 631 and between the electrode tube 83 and the current source 631. The invention is not limited to connect the electrode tube 83 to the connecting tube 80, and a conductive film (for example, a metallic film) provided on an inner peripheral surface of the connecting tube 80 may be used as electrodes to be paired with the electrode members 75 and 82.

In the fluid transport apparatus 2 having the configuration as described above, the current source 631 supplies a constant current to the electrode tube 83, and the potential measuring portion 632 measures the potential between the electrode member 82 of the connecting tube 80 and the electrode tube 83, so that the trouble determining unit 64 may determine the troubles of the catheter 71 and the connecting tube 80 on the basis of the result of measurement of the potential thereof. Specifically, in the case where the catheter 71 and the connecting tube 80 are normal, as illustrated in Fig. 7, the closed current path is defined by the current source 631, the electrode tube 83, the fluid A flowing in the interior of the connecting tube 80, the connecting needle 81, the port base 73, and the catheter 71, the biological body B, the electrode member 75 of the catheter 71, and the electrode member 82 of the connecting tube 80, where a predetermined current flows therein. Therefore, a potential within a predetermined range in accordance with the current from the current source 631 is measured. In contrast, if there is a trouble such as coming away or clogging of the catheter 71 or the connecting tube 80, the closed current path via the fluid A flowing in the interior of the catheter 71 and the connecting tube 80 and the biological body B may not be formed, or the predetermined current may not be flowed to the electrode members 75 and 82, so that a potential deviated from the predetermined range may be measured. Therefore, the trouble determining unit 64 may determine the trouble of the catheter 71 and the connecting tube 80 on the basis of the measured potential.

### Fifth Embodiment

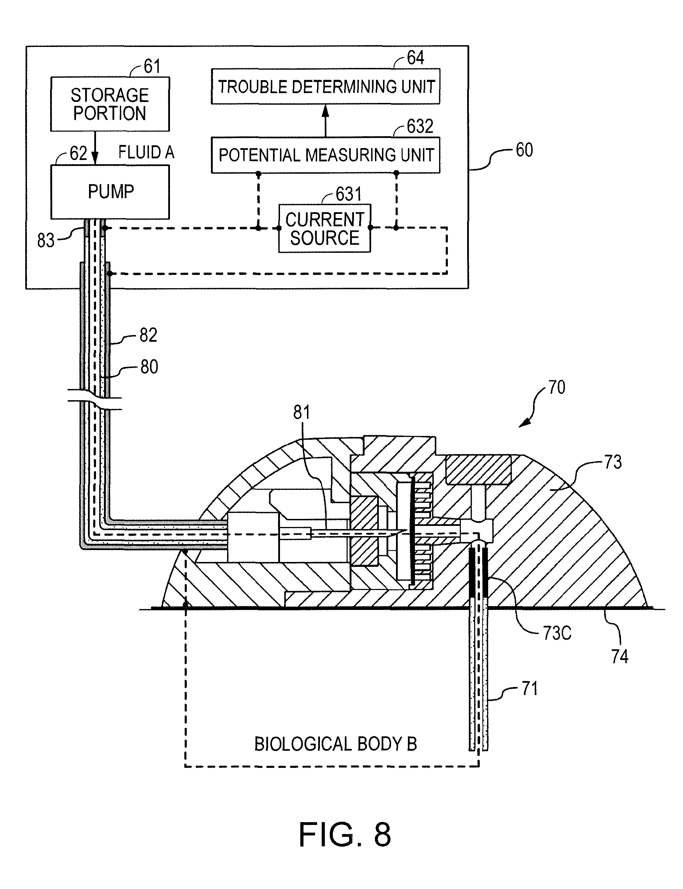

Fig. 8 is an explanatory drawing of a trouble determining method for the catheter 71 and the connecting tube 80 of a fifth embodiment. The fluid transport apparatus of the fifth embodiment is the fluid transport apparatus 2 in which the main body portion 60 and the fluid infusing portion 70 are connected with the elongated connecting tube 80 in the same manner as the fourth embodiment. However, as illustrated in Fig. 8, the connecting tube 80 is provided with the electrode member 82, which does not come into contact with the fluid, but the catheter 71 is not provided with the electrode member. Instead, in the fifth embodiment, the adhesive pad 74 configured to come into contact with and be adhered to the biological body is used as a conductive pad. Then, the adhesive pad 74 and the electrode member 82 of the connecting tube 80 are electrically connected, and the electrode member 82 and the current source 631 are electrically connected. In contrast, the electrode tube 83 (second electrode) configured to come into contact with the fluid is connected to the end portion of the connecting tube 80 on the main body portion 60 side to electrically connect the electrode tube 83 and the current source 631. The potential measuring portion 632 is electrically connected between the electrode member 82 and the current source 631 and between the electrode tube 83 and the current source 631. The invention is not limited to provide the entire surface of the adhesive pad 74 with conductivity, but the adhesive pad 74 may be partly provided with conductivity. In this case, by providing a center portion of the adhesive pad 74 with conductivity, the influence on the potential measurement is reduced even though the periphery of the adhesive pad 74 is separated. In addition, by employing the conductive pad as the adhesive pad 74, an increase in the number of components is prevented. However, the invention is not limited thereto, and an electrode configured to come into contact with the biological body may be provided on a lower surface of the adhesive pad 74.

In the fluid transport apparatus 2 having the configuration as described above, the current source 631 supplies a constant current to the electrode tube 83, and the potential measuring portion 632 measures the potential between the electrode member 82 of the connecting tube 80 and the electrode tube 83, so that the trouble determining unit 64 may determine the trouble of the catheter 71 and the connecting tube 80 on the basis of the result of measurement of the potential thereof. Specifically, in the case where the catheter 71 and the connecting tube 80 are normal, as illustrated in Fig. 8, the closed current path is defined by the current source 631, the electrode tube 83, the fluid A flowing in the interior of the connecting tube 80, the connecting needle 81, the port base 73, and the catheter 71, the biological body B, the adhesive pad 74, and the electrode member 82 of the connecting tube 80, as illustrated in Fig. 7, where a predetermined current flows therein. Therefore, a potential within a predetermined range in accordance with the current from the current source 631 is measured. In contrast, if there is a trouble such as coming away or clogging of the catheter 71 or the connecting tube 80, the closed current path via the fluid A flowing in the catheter 71 and the connecting tube 80, and the biological body B may not be formed, or a predetermined current may not be flowed to the electrode member 82, so that a potential deviated from a predetermined range may be measured. Therefore, the trouble determining unit 64 may determine the trouble of the catheter 71 and the connecting tube 80 on the basis of the measured potential.

### Sixth Embodiment

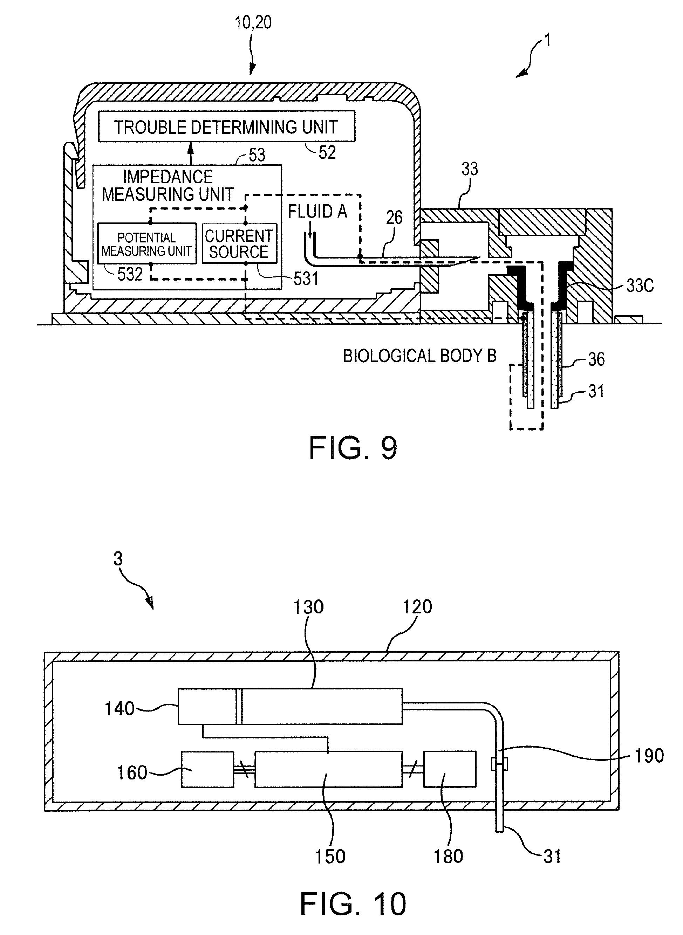

Fig. 9 is an explanatory drawing of a trouble determining method for the catheter 31 of a sixth embodiment. In Fig. 9, the fluid transport apparatus 1 having almost the same configuration as the fluid transport apparatus 1 of the first embodiment is exemplified. In the first to the fifth embodiments, the trouble determining units 52 and 64 determine the troubles of the catheters 31 and 71 and the connecting tube 80 on the basis of the potential measured by the potential measuring units 512 and 632. However, the invention is not limited thereto. In this case, as illustrated in Fig. 9, an impedance measuring unit 53 including a current source 531 and a potential measuring unit 532, and the trouble determining unit 52 may be provided on a control unit (not illustrated) provided on the main body portion 10 of the fluid transport apparatus 1. In this case, the current source 531 supplies a constant current to the connecting needle 26, and the potential measuring unit 532 measures the potential between the connecting needle 26 and the electrode member 36 of the catheter 31, so that the impedance measuring unit 53 is capable of obtaining impedances on the basis of the measured potential and the known current value supplied by the current source 531. On the basis of the result of the impedance, the trouble determining unit 52 may determine the trouble of the catheter 31. Specifically, when the catheter 31 is normal, the closed current path is defined by the current source 531, the connecting needle 26, the fluid A, the biological body B, and the electrode member 36 of the catheter 31, a predetermined current flows therethrough, and a potential within a predetermined range is measured, so that the impedance within the predetermined range is obtained. In contrast, if there is the trouble in the catheter 31, the closed current path via the fluid A flowing in the catheter 31 and the biological body B may not be formed, or the predetermined current may not be flowed to the electrode member 36, so that a impedance deviated from the predetermined range may be measured. Therefore, the trouble determining unit 52 is capable of determining the trouble of the catheter 31 on the basis of the impedance obtained by the impedance measuring unit 53. In Fig. 9, the fluid transport apparatus 1 of the first embodiment is exemplified. However, in the fluid transport apparatuses 1 and 2 of the second to the fifth embodiments as well, the impedance measuring unit may be provided instead of the potential measuring units 532 and 632, and the troubles of the catheters 31 and 71 or the connecting tube 80 may be determined on the basis of the impedance.

### Seventh Embodiment

In the embodiments described above, the fluid is pumped by the pump of the type compressing the tube 21 by the plurality of fingers 22 in sequence. However, the type of the pump is not limited thereto.

Fig. 10 is a cross-sectional view of a fluid transport apparatus 3 of a seventh embodiment. In Fig. 10, the fluid transport apparatus 3 including a reservoir 130, a dispenser 140, a control unit 150 including a local processor or the like, a wireless receiver 160, an electric source 180, and a tube 190 stored in the housing 120 is illustrated.

Fluid to be administered to the biological body or the like is stored in the reservoir 130. The dispenser 140 is configured to apply a pressure to the drug solution by changing the capacity in the reservoir 130, and cause the fluid to flow to the tube 190 and the catheter 31.

The wireless receiver 160 receives an instruction from a remote control apparatus, which is not illustrated. Then, the instruction is sent to the control unit 150. The control unit 150 controls the dispenser 140 and applies a pressure to the fluid in the reservoir 130 as described above. The electric source 180 supplies power to the wireless receiver 160 and the control unit 150.

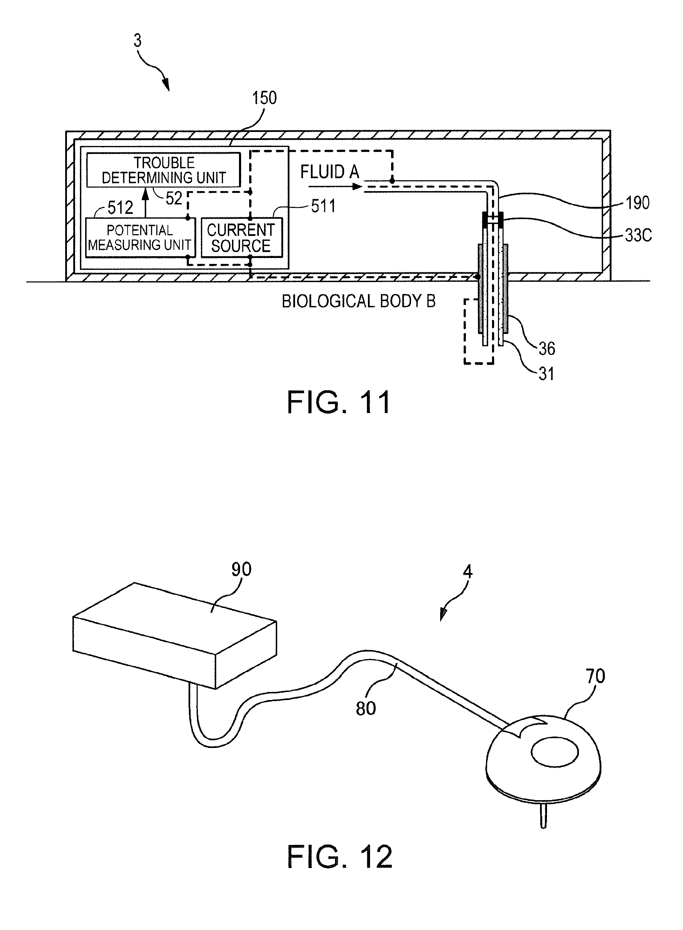

Fig. 11 is an explanatory drawing of a trouble determining method for the catheter 31 of the seventh embodiment. In the seventh embodiment, the determination of the trouble of the catheter 31 is performed by using almost the same principle as the first embodiment described above. In Fig. 11, the same configurations as the first embodiment described above are denoted by the same reference numerals and description thereof will be omitted.

As illustrated in Fig. 11, the control unit 150 may be provided with the trouble determining unit 52, the current source 511, and the potential measuring unit 512. As illustrated in Fig. 11, the catheter 31 the electrode member 36, and the coupling portion 33C may be provided. Accordingly, even though a pump unit configured to cause fluid to flow has a configuration as the seventh embodiment in the fluid transporting apparatus 3, whether or not the catheter 31 is normal may be determined by using the same principle as the first embodiment.

In the seventh embodiment, the impedance measuring unit may be provided instead of the potential measuring unit 512 as in the sixth embodiment described above and the trouble of the catheter 31 may be determined on the basis of the impedance.

### Eighth Embodiment

Fig. 12 is an explanatory drawing of a fluid transport apparatus 4 of an eighth embodiment. Fig. 12 illustrates a main body portion 90, the fluid infusing portion 70, and the connecting tube 80. The fluid infusing portion 70 and the connecting tube 80 in Fig. 12 are the same as that illustrated in Fig. 6A in the fourth embodiment described above. The main body portion 90 in Fig. 12 is substantially the same as the fluid transport apparatus 3 of the seventh embodiment, but is different in that the catheter 31 is not provided, the connecting tube 80 also serves as the tube 190, and is connected to the fluid infusing portion 70 via the connecting tube 80.

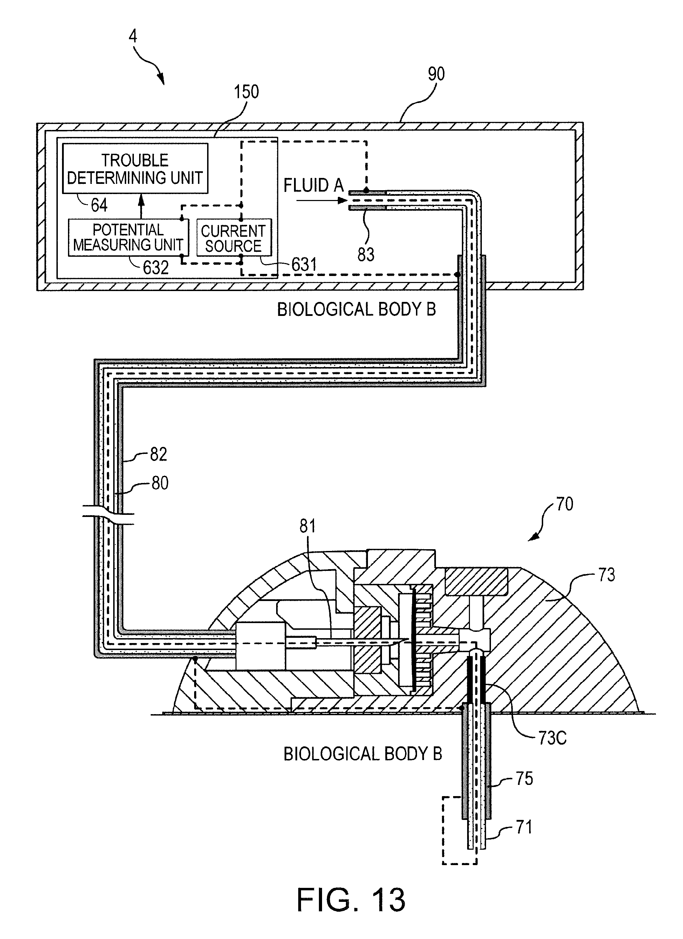

Fig. 13 is an explanatory drawing of a trouble determining method for the catheter 71 and the connecting tube 80 of the eighth embodiment. In the eighth embodiment, the determination of the troubles of the catheter 71 and the connecting tube 80 is performed by using almost the same principle as in the fourth embodiment described above. In Fig. 13, the same configurations as the fourth embodiment described above are denoted by the same reference numerals and description will be omitted.

As illustrated in Fig. 13, the control unit 150 of the main body 90 may be provided with the trouble determining unit 64, the current source 631, and the potential measuring portion 632. As illustrated in Fig. 13, the connecting tube 80 and the electrode member 82 may be provided. In this configuration, whether or not the catheter 71 and the connecting tube 80 are normal may be determined by using the same principle as the fourth embodiment.

In the eighth embodiment as well, the impedance measuring unit may be provided instead of the potential measuring unit 632 as in the sixth embodiment, and the troubles of the catheter 71 or the connecting tube 80 may be determined on the basis of the impedance.

### Ninth Embodiment

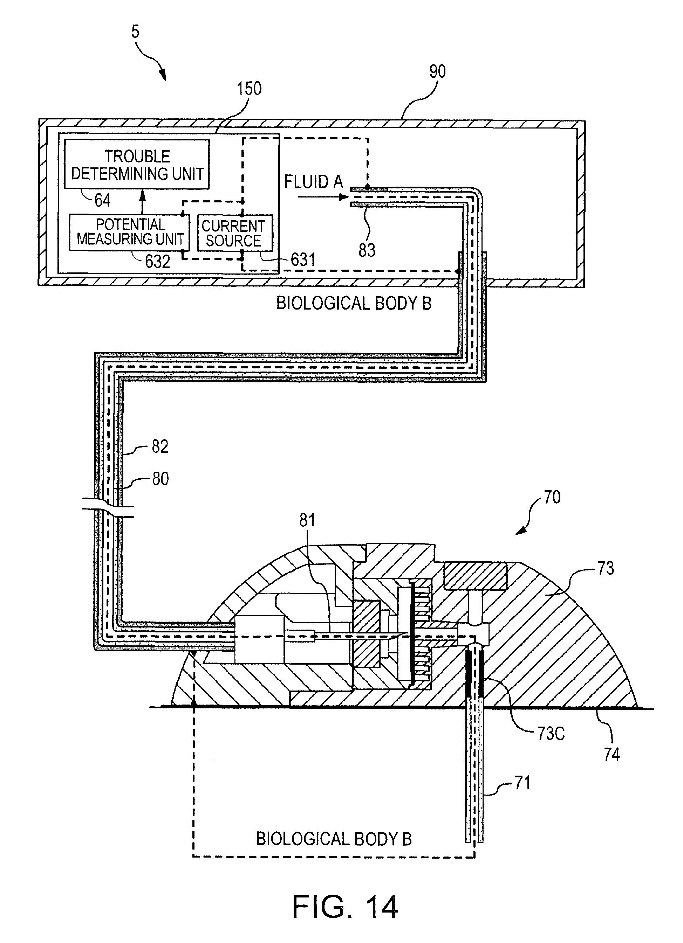

Fig. 14 is an explanatory drawing of a trouble determining method for the catheter 71 and the connecting tube 80 of a ninth embodiment. In the ninth embodiment, the determination of the troubles of the catheter 71 and the connecting tube 80 is performed by using almost the same principle as in the fifth embodiment described above. In Fig. 14, the same configurations as the fifth embodiment described above are denoted by the same reference numerals and description will be omitted.

As illustrated in Fig. 14, the control unit 150 of the main body portion 90 may be provided with the trouble determining unit 64, the current source 631, and the potential measuring portion 632. In this configuration, whether or not the catheter 71 and the connecting tube 80 are normal may be determined by using the same principle as the fifth embodiment.

In the ninth embodiment as well, the impedance measuring unit may be provided instead of the potential measuring unit 632 as in the sixth embodiment, and the troubles of the catheter 71 and the connecting tube 80 may be determined on the basis of the impedance.

### Other Embodiments

The fluid transport apparatus descried above may also be referred to as a micro pump. The fluid transport apparatus described above is also referred to a fluid administering apparatus. The tube used in the micro pump is referred to as cannula.

The embodiments described above are for facilitating the understanding of the invention, and are not for interpreting the invention in a limited range. It is needless to say that the invention may be modified or improved without departing from the scope of the invention and equivalents are included in the invention. For example, the fluid transport apparatus descried in the above-described embodiment is provided with the pump configured to transport the fluid. However, the invention is not limited thereto and, for example, the fluid transport apparatus which is not provided with the pump and is configured to transport the fluid by utilizing the height difference between the position of the fluid storage portion and the position of the catheter is also applicable.

## Claims

**1.** A fluid transport apparatus comprising:
an insulative transporting tube that constitutes part of a flow channel in which conductive fluid is transported;
a first electrode provided on the transporting tube and configured not to come into contact with the fluid in the flow channel;
a second electrode configured to come into contact with the fluid in the flow channel; and
a determining unit connected to the first electrode and the second electrode and configured to determine a trouble of the transporting tube.

**2.** The fluid transport apparatus according to Claim 1, wherein
the determining unit determines whether or not a current path is formed between the first electrode and the second electrode.

**3.** The fluid transport apparatus according to Claim 1 or 2, further comprising:
a power source connected between the first electrode and the second electrode and configured to apply a current between the first electrode and the second electrode.

**4.** The fluid transport apparatus according to any one of the preceding claims, further comprising:
a potential measuring unit configured to measure a potential between the first electrode and the second electrode, wherein the determining unit determines the trouble of the transporting tube on the basis of the potential.

**5.** The fluid transport apparatus according to any one of the preceding claims, further comprising:
a notifying unit configured to notify the trouble of the transporting tube to a user.

**6.** The fluid transport apparatus according to any one of the preceding claims, wherein
the transporting tube is a catheter configured to be inserted into the biological body in order to infuse the fluid into the biological body.

**7.** The fluid transport apparatus according to any one of the preceding claims, wherein
the first electrode is provided on a portion of an outer peripheral surface of the catheter other than an end portion on a side to be inserted into the biological body.

**8.** The fluid transport apparatus according to any one of the preceding claims, wherein
the transporting tube is a connecting tube configured to connect a fluid storage portion in which the fluid is stored and a fluid infusing portion provided with the catheter to be inserted into the biological body for infusing the fluid into the biological body.

**9.** The fluid transport apparatus according to any one of the preceding claims, wherein
the second electrode is a conductive film provided on an inner peripheral surface of the transporting tube or a conductive tube connected to the transporting tube and which constitutes part of the flow channel.

**10.** The fluid transport apparatus according to any one of the preceding claims, wherein
the first electrode is provided on an outer peripheral surface of the transporting tube, part of the outer peripheral surface of the transporting tube is exposed, and the fluid flowing into the interior of the transporting tube is visible.

**11.** The fluid transport apparatus according to any one of the preceding claims, wherein
the first electrode is embedded in the transporting tube.

**12.** The fluid transporting apparatus according to any one of the preceding claims, wherein
the flow channel is provided with a pump configured to cause the fluid to flow.

**13.** The fluid transport apparatus according to Claim 12, wherein
the pump causes the fluid to flow by compressing the tube in which the fluid flows with a plurality of pressing members in sequence.
→ "Further embodiments"

**16.** A trouble determining method for a transporting tube, comprising
causing current to flow between a first electrode provided on an insulating transporting tube which constitutes part of a flow channel in which conductive fluid is transported and configured not to come into contact with the fluid in the flow channel, and a second electrode configured to come into contact with the fluid in the flow channel, and
determining a trouble of the transporting tube on the basis of the state of the measured current.
